# EUROPÄISCHE PATENTSCHRIFT

(11) **EP 1 230 205 B1**
(45) Veröffentlichungstag und Bekanntmachung des Hinweises auf die Patenterteilung: **08.12.2004**
(21) Anmeldenummer: 00969152.8
(22) Anmeldetag: 31.10.2000
(51) Int. Cl.: C07C 51/41, C07C 53/128, A61K 31/19, A61P 25/06, A61P 25/08

(54) **VERFAHREN ZUR HERSTELLUNG VON VERBINDUNGEN DER VALPROINSÄURE**
METHOD FOR PRODUCING COMPOUNDS OF THE VALPROINIC ACID
PROCEDE DE PRODUCTION DE COMPOSES D'ACIDE VALPROIQUE

(30) Priorität: 02.11.1999 CH 199799
(43) Veröffentlichungstag der Anmeldung: 14.08.2002
(73) Patentinhaber: Cilag AG, 8205 Schaffhausen (CH)
(72) Erfinder: WEH, Christian, 88175 Scheidegg (DE)
(74) Vertreter: Braun, André, jr.
(86) Internationale Anmeldenummer: PCT/CH2000/000578
(87) Internationale Veröffentlichungsnummer: WO 2001/032595

(56) Entgegenhaltungen:
- WO-A-81/00562
- DD-A- 215 533
- US-A- 4 895 873
- US-A- 5 017 613
- US-A- 5 795 615

## Beschreibung

Die vorliegende Erfindung betrifft ein Verfahren zur Herstellung von oligomeren Verbindungen der Valproinsäure, insbesondere ein Verfahren für die Herstellung von ausgewählten oligomeren Verbindungen der Valproinsäure, ohne Zugabe eines Lösungsmittels. Valproinsäure wird auch als 2-Propylpentansäure, als 2-Propylvaleriansäure oder als Di-n-propyl-essigsäure bezeichnet. Im weiteren wird der Ausdruck Valproinsäure verwendet.

Valproinsäure sowie oligomere Verbindungen sind an sich bekannt. Valproinsäure, Valproinsäure-natriumsalz (Natriumvalproat) und die oligomere 1:1 Verbindung von Natriumvalproat und Valproinsäure, das sogenannte Divalproex-natriumsalz, sind Wirkstoffe zur medizinischen Behandlung von epileptischen Anfällen, Krämpfen und Migräne. Valproinsäure ist bei Raumtemperatur flüssig und deshalb für die Herstellung fester Arzneimittelformulierungen, wie beispielsweise Tabletten, nicht geeignet. Natriumvalproat ist bei Raumtemperatur fest, jedoch ist es sehr hygroskopisch, was die Herstellung von festen Arzneimittelformulierungen zur oralen Verabreichung sehr erschwert. Divalproex-natriumsalz ist weniger hygroskopisch, doch zeigt die Verbindung bei längerer Lagerung eine Tendenz zur Verklumpung und Verkrustung. Die nachfolgend beschriebenen oligomeren Verbindungen der Valproinsäure mit unterschiedlichen Stöchiometrien und Solvaten stellen Möglichkeiten zur Formulierung der Valproinsäure dar, welche die genannten Nachteile nicht oder in erheblich reduziertem Mass aufweisen. Es besteht deshalb ein Interesse, solche Verbindungen möglichst einfach herstellen zu können. DD-A-215 533 offenbart die Herstellung von Alkali- und Erdalkalisalzen der Di-n-propylessigsäure unter Verwendung von organischen Lösungsmitteln. US-A-5 017 613 betrifft die galenische Herstellung einer pharmazeutischen Zusammensetzung auf der Basis einer Kombination von Valproinsäure und eines pharmazeutisch annehmbaren Salzes von Valproinsäure. Insbesondere wurde gefunden, dass oligomeren Verbindungen von Natriumvalproat und Valproinsäure ohne Zugabe eines Lösungsmittels zum Reaktionsgemisch hergestellt werden können, was ökologisch und ökonomisch vorteilhaft ist. Das erfindungsgemässe Verfahren hat auch den Vorteil, dass langwierige und energieintensive Trocknungsprozesse vermieden werden und umweltrelevante Aspekte, wie beispielsweise Resourcenminimierung, Einsparung von Rohstoffen und Energie oder Abfallverminderung berücksichtigt werden können. Insbesondere ermöglicht das erfindungsgemässe Verfahren, die Wirkstoffe ohne Trocknen, schonend und unter Vermeidung temperaturbedingter Zersetzungsvorgänge herzustellen. Gleichzeitig bietet das erfindungsgemässe Verfahren eine Chance für Ärzte und Patienten, aus der Vielzahl der verschiedenen gleichermassen hochwertigen Verbindungen der Valproinsäure und der Salze der Valproinsäure, den Wirkstoffen ihrer Präferenz auszuwählen, welche alle mittels eines an sich identischen Herstellungsprozesses hergestellt wurden. Ein besonderer Vorteil der vorliegenden Erfindung ist es, dass mit dem erfindungsgemässen Verfahren Verbindungen mit ausgewählten Stöchiometrien, d.h. mit ausgewählten Verhältnissen von Valproinsäuresalz und Valproinsäure, hergestellt werden können, was bis anhin für diese Zusammensetzungen mittels Kristallisation aus organischen Lösungsmitteln nicht möglich gewesen ist. Ein weiterer Vorteil des erfindungsgemässen Verfahrens liegt darin, dass die Verwendung von Natriumvalproat als hygroskopische Natriumquelle entfällt.

Die vorliegende Erfindung ist in den Patentansprüchen definiert. Insbesondere betrifft die vorliegende Erfindung ein Verfahren zur Herstellung von Verbindungen, welche mindestens ein Molekül Valproinsäuresalz und mindestens ein Molekül Valproinsäure enthalten, und das Valproinsäuresalz ein Alkali- oder Erdalkalisalz darstellt, welches dadurch gekennzeichnet ist, dass man Valproinsäure, ohne Zugabe eines Lösungsmittels, bei einer Temperatur, die höher ist als die Schmelztemperatur der Valproinsäure, direkt mit der berechneten Menge des entsprechenden Alkalikarbonats oder Erdalkalikarbonats und/oder der berechneten Menge des entsprechenden Alkalibikarbonats oder Erdalkalibikarbonats, umsetzt.

Dabei beträgt die Reaktionstemperatur vorzugsweise 50°C bis 250°C, vorzugsweise 70 °C bis 180 °C, wobei das in der Reaktion sich bildende Kohlenstoffdioxid und Wasser fortwährend aus dem Reaktionsgemisch entfernt werden. Dabei reagiert die Valproinsäure direkt und vollständig mit dem Karbonat (z.B. Na₂CO₃, CaCO₃) oder dem Bikarbonat (z.B. LiHCO₃, Ca(HCO₃)₂) unter Bildung von CO₂ und Wasser. Soll die Verbindung als Hydrat hergestellt werden, so wird vorzugsweise nach Beendigung der Umsetzung die berechnete Menge Wasser dem Produkt zugesetzt.

Das Valproinsäuresalz als Alkalisalz ist vorzugsweise ein Salz von Lithium, Natrium, Kalium oder Rubidium, vorzugsweise von Natrium oder Kalium. Das Valproinsäuresalz als Erdalkalisalz ist vorzugsweise ein Salz von Magnesium, Kalzium, Strontium oder Barium, vorzugsweise von Magnesium oder Kalzium.

Die Verbindungen gemäss der vorliegenden Erfindung, welche mindestens ein Molekül Valproinsäuresalz und mindestens ein Molekül Valproinsäure enthalten, entsprechen der allgemeinen Formel (I):

[(CH₃CH₂CH₂)₂CH-C(O)OMe]ₘ . [(CH₃CH₂CH₂)₂CH-C(O)ₙ . xH₂O (I)

worin
- Me: Li⁺, Na⁺, K⁺, Rb⁺, Mg⁺², Ca⁺², Sr⁺² oder Ba⁺², vorzugsweise Na⁺, K⁺, Mg⁺², Ca⁺²,
- m: eine ganze Zahl von 1 bis 10, vorzugsweise von 1 bis 6,
- n: eine ganze Zahl von 1 bis 9, vorzugsweise von 1 bis 3, und das Verhältnis von m : n von 1:1 bis 6:1, vorzugsweise 1 : 1 bis 5 : 3 vorzugsweise 1 : 1 oder 4 : 3 oder 2 : 1 und
- x: Null, 1 oder 2, vorzugsweise null oder 1, bedeuten.

In den folgenden Beispielen geben die Zahlen in den Klammern jeweils das Verhältnis von (m+n:m) an. Beispiele von Verbindungen der Formel (I) sind: 2-Propylpentansäure (2:1) natriumsalz; 2-Propylpentansäure (2:1) natriumsalz-monohydrat sowie das Dihydrat; 2-Propylpentansäure (3:2) natriumsalz; 2-Propylpentansäure (4:3) natriumsalz; 2-Propylpentansäure (4:3) natriumsalz-monohydrat; 2-Propylpentansäure (5:3) natriumsalz; 2-Propylpentansäure(7:6)natriumsalz, sowie jeweils das Monohydrat und das Dihydrat; vorzugsweise
2-Propylpentansäure(m+n:m)natriumsalz-xH₂O, worin m+n eine ganze Zahl von 3 bis 10, m jeweils 1 bis (m+n-1), und x Null, eins oder zwei bedeuten;
2-Propylpentansäure (2:1) lithiumsalz; 2-Propylpentansäure-(2:1) lithiumsalz-monohydrat sowie das Dihydrat; 2-Propylpentansäure(4:3)lithiumsalz; 2-Propylpentansäure(4:3)-lithiumsalz-monohydrat; vorzugsweise
2-Propylpentansäure (m+n:m)lithiumsalz-xH₂O, worin m+n eine ganze Zahl von 2 bis 10, m jeweils 1 bis (m+n-1), und x Null, eins oder zwei bedeuten;
2-Propylpentansäure(2:1)kalium alz; 2-Propylpentansäure-(2:1) kaliumsalz-monohydrat; 2-Propylpentansäure (3:2) kaliumsalz; 2-Propylpentansäure (4:3) kaliumsalz-monohydrat; vorzugsweise 2-Propylpentansäure (m+n:m) kaliumsalz-XH₂O, worin m+n eine ganze Zahl von 2 bis 10, m jeweils 1 bis (m+n-1), und x Null, eins oder zwei bedeuten;
2-Propylpentansäure (2:1)rubidiumsalz; 2-Propylpentansäure-(2:1) rubidiumsalz-monohydrat; 2-Propylpentansäure (3:2) rubidiumsalz; 2-Propylpentansäure (4:3) rubidiumsalz-monohydrat;
2-Propylpentansäure(m+n:m) rubidiumsalz-xH₂O, worin m+n eine ganze Zahl von 2 bis 10, m jeweils 1 bis (m+n-1), und x Null, eins oder zwei bedeuten; vorzugsweise
2-Propylpentansäure (2:1)magnesiumsalz; 2-Propylpentansäure(2:1)magnesiumsalz-monohydrat; 2-Propylpentansäure-(3:2)magnesiumsalz; 2-Propylpentansäure(4:3)magnesiumsalzmonohydrat; vorzugsweise
2-Propylpentansäure(m+n:m)magnesiumsalz-xH₂O, worin m+n eine ganze Zahl von 3 bis 10, m jeweils 1 bis (m+n-1),und x Null, eins oder zwei bedeuten;
2-Propylpentansäure (2:1) kal ziumsalz; 2-Propylpentansäure-(2:1) kalziumsalz-monohydrat; 2-Propylpentansäure (3:2) kalziumsalz; 2-Propylpentansäure (4:3) kalziumsaiz-monohydrat; vorzugsweise 2-Propylpentansäure(m+n:m)kalziumsalz-xH₂O,
worin m+n eine ganze Zahl von 3 bis 10, m jeweils 1 bis (m+n-1), und x Null, eins oder zwei bedeuten.
2-Propylpentansäure(2:1)strontiumsalz; 2-Propylpentansäure(2:1)strontiumsalz-monohydrat; 2-Propylpentansäure-(3:2)strontiumsalz; 2-Propylpentansäure (4:3)strontiumsalz-monohydrat; vorzugsweise
2-Propylpentansäure(m+n:m)strontiumsalz-xH₂O, worin m+n eine ganze Zahl von 2 bis 10, m jeweils 1 bis (m+n-1), und x Null, eins oder zwei bedeuten;
2-Propylpentansäure (2:1)bariumsalz; 2-Propylpentansäure-(2:1)bariumsalz-monohydrat; 2-Propylpentansäure(3:2)bariumsalz; 2-Propylpentansäure(4:3)bariumsalz-monohydrat; vorzugsweise 2-Propylpentansäure (m+n:m)bariumsalz-xH₂O, worin m+n eine ganze Zahl von 2 bis 10, m jeweils 1 bis (m+n-1), und x Null, eins oder zwei bedeuten.

Die folgenden Beispiele erläutern die Erfindung.

### Beispiel 1

In einem Rundkolben, ausgerüstet mit Rührwerk, Rückflusskühler und Heizung werden 144.21 g (1 Mol) flüssige Valproinsäure sowie ein Alkalikarbonat oder Erdalkalikarbonat gemäss den in Tabelle 1 angegebenen Mengen vorgelegt. Das Gemisch wird gerührt und langsam auf über 300°C (>100 °C) erwärmt, wobei die Reaktion unter Bildung von CO₂ und Wasser einsetzt. Das sich bildende CO₂ und das Wasser werden laufend entfernt, wobei das CO₂ gasförmig entweicht und das sich bildende Wasser über eine absteigende Kolonne destilliert. Mit Beenden der Umsetzung wird ein klares Produkt erhalten, welches über ein Schmelzblech gewonnen wird. Der Wassergehalt des erhaltenen Produktes wird nach der Methode von Karl-Fischer bestimmt und die fehlende Menge Wasser für die Herstellung eines bestimmten Hydrates ergänzt.

**Tabelle 1**

| Versuchs Nr. | Valproinsäure (Gramm, Mol) | Eingesetztes Karbonat/Bikarbonat (Gramm, Grammäquivalente) | Wasser (Gramm, Grammäquivalente) | (m+n):m |
|---|---|---|---|---|
| 1 | 144,21 g,1.0 Mol | Na₂CO₃ (35.33g, 0.33mol) | 0 | 3:2 |
| 2 | 144,21 g,1.0 Mol | Na₂CO₃ (31.80g, 0.30mol) | 0 | 5:3 |
| 3 | 144,21 g,1.0 Mol | Na₂CO₃ (39.75g, 0.38mol) | (4.50g, 0.25mol) | 4:3 |
| 4 | 144,21 g,1.0 Mol | K₂CO₃ (34.55g, 0.25mol) | 0 | 2:1 |
| 5 | 144,21 g,1.0 Mol | Rb₂CO₃ (57.74g, 0.25mol) | 0 | 2:1 |
| 6 | 144,21 g,1.0 Mol | NaHCO₃ (42.01g, 0.50mol) | 0 | 2:1 |

Die Muster wurden mittels Elementaranalyse bestimmt. Im weiteren wurde die Phasenanalyse mittels Röntgendiffraktometer-Aufnahmen (XRD) an Pulverpräparaten bestimmt.
Beispiele solcher Analysen sind:
2-Propylpentansäure(4:3)natriumsalz-monohydrat (OMNIVAL I)
   Trikline Elementarzelle: Figure of Merit M = 12.6
   aₒ = 15.9 bₒ = 13.5 cₒ = 5.4
   alpha = 101° beta = 97° gamma = 111°
   Zellvolumen 1025
   5 Moleküle in der Elementarzelle ergeben eine Röntgendichte von 1.27 g/cm³
2-Propylpentansäure(3:2)natriumsalz (OMNIVAL II) :
   Trikline Elementarzelle: Figure of Merit M = 5.4
   aₒ = 15.5 bₒ = 13.0 cₒ = 10.4
   alpha = 89° beta = 93° gamma = 110° Zellvolumen 1973
   9 Moleküle in der Elementarzelle ergeben eine Röntgendichte von 1.20 g/cm³

## Patentansprüche

1. Verfahren zur Herstellung von Verbindungen, welche mindestens ein Molekül Valproinsäuresalz und mindestens ein Molekül Valproinsäure enthalten, und das Valproinsäuresalz ein Alkali- oder Erdalkalisalz darstellt,
**dadurch gekennzeichnet, dass** man Valproinsäure, ohne Zugabe eines Lösungsmittels, bei einer Temperatur, die höher ist als die Schmelztemperatur der Valproinsäure, direkt mit der berechneten Menge des entsprechenden Alkalikarbonats oder Erdalkalikarbonats und/oder der berechneten Menge des entsprechenden Alkalibikarbonats oder Erdalkalibikarbonats, umsetzt.

2. Verfahren nach Anspruch 1, **dadurch gekennzeichnet, dass** die Reaktionstemperatur 50°C bis 250°C, vorzugsweise 70 °C bis 180 °C, beträgt, wobei das in der Reaktion sich bildende Kohlenstoffdioxid und das Wasser fortwährend aus dem Reaktionsgemisch entfernt werden.

3. Verfahren nach Anspruch 1 oder 2, **dadurch gekennzeichnet, dass** ein Hydrat hergestellt wird, wobei nach Beendigung der Umsetzung die berechnete Menge Wasser dem Produkt zugesetzt wird.

4. Verfahren nach einem der Ansprüche 1-3, **dadurch gekennzeichnet, dass** das Valproinsäuresalz als Alkalisalz ein Salz von Lithium, Natrium, Kalium oder Rubidium, vorzugsweise von Natrium oder Kalium darstellt.

5. Verfahren nach einem der Ansprüche 1-3, **dadurch gekennzeichnet, dass** das Valproinsäuresalz als Erdalkalisalz ein Salz von Magnesium, Kalzium, Strontium oder Barium, vorzugsweise von Magnesium oder Kalzium darstellt.

6. Verfahren nach einem der Ansprüche 1-5, **dadurch gekennzeichnet, dass** eine Verbindung der allgemeinen Formel (I):
[(CH₃CH₂CH₂)₂CH-C(O)OMe]ₘ . [(CH₃CH₂CH₂)₂CH-C(O)OH]ₙ . xH₂O (I)
hergestellt wird, worin
Me Li⁺, Na⁺, K⁺, Rb⁺, Mg⁺², Ca⁺², Sr⁺² oder Ba⁺², vorzugsweise Na⁺, K⁺, Mg⁺², Ca⁺²,
m eine ganze Zahl von 1 bis 10, vorzugsweise von 1 bis 6,
n eine ganze Zahl von 1 bis 9, vorzugsweise von 1 bis 3,
und das Verhältnis von m : n von 1:0.05 bis 6:1, vorzugsweise 1 : 1 bis 5 : 3 vorzugsweise 1 : 1 oder 4 : 3 oder 2 : 1 und
x Null, 1 oder 2, vorzugsweise null oder 1,
bedeuten.

7. Verfahren nach einem der Ansprüche 1-6, **dadurch gekennzeichnet, dass** die Verbindung 2-Propylpentansäure(3:2)natriumsalz herstellt wird.

8. Verfahren nach einem der Ansprüche 1-6, **dadurch gekennzeichnet, dass** die Verbindung 2-Propylpentansäure (4: 3) natriumsalz-monohydrat hergestellt wird.

## Claims

1. Method of preparing compounds containing at least one molecule of valproic acid salt and at least one molecule of valproic acid, and wherein the valproic acid salt is an alkali- or an alkaline earth metal salt, **characterized in that** valproic acid is reacted directly with the calculated amount of the corresponding alkali metal carbonate or alkaline earth metal carbonate and/or with the calculated amount of the corresponding alkali metal bicarbonate or alkaline earth metal bicarbonate, without the addition of a solvent and at a temperature above the melting point of the valproic acid.

2. Method according to claim 1, **characterized in that** the reaction temperature is from 50°C to 250°C, preferably 70°C to 180°C, and wherein the carbon dioxide and the water, which are formed during the reaction, are continuously removed from the reaction mixture.

3. Method according to claim 1 or 2, **characterized in that** a hydrate is prepared comprising the step of adding the calculated amount of water to the product after the reaction has been completed.

4. Method according to any one of the claims 1-3, **characterized in that** the valproic acid salt as an alkali metal salt is a salt of lithium, sodium, potassium or rubidium, preferably of sodium or potassium.

5. Method according to any one of the claims 1-3, **characterized in that** the valproic acid salt as an alkaline earth metal salt is a salt of magnesium, calcium, strontium or barium, preferably of magnesium or calcium.

6. Method according to any one of the claims 1-5, **characterized in that** a compound of general formula (I):
[(CH₃CH₂CH₂)₂CH-C(O)OMe]ₘ . [(CH₃CH₂CH₂)₂ CH-C(O)OH]ₙ . xH₂O (I)
is prepared in which
Me is Li⁺, Na⁺, K⁺, Rb⁺, Mg²⁺, Ca²⁺, Sr²⁺ or Ba²⁺, preferably Na⁺, K⁺, Mg²⁺, Ca²⁺,
m is an integer from 1 to 10, preferably from 1 to 6,
n is an integer from 1 to 9, preferably from 1 to 3,
and the ratio of m:n is from 1:0.05 to 6:1, preferably 1:1 to 5:3, preferably 1:1 or 4:3 or 2: 1, and
x is zero, 1 or 2, preferably zero or 1.

7. Method according to any one of the claims 1-6, **characterized in that** the compound 2-propylpentanoic acid(3:2)sodium salt is prepared.

8. Method according to any one of the claims 1-6, **characterized in that** the compound 2-propylpentanoic acid (4:3) sodium salt monohydrate is prepared.

## Revendications

1. Procédé pour la fabrication de composés contenant au moins une molécule de sel d'acide valproïque et au moins une molécule d'acide valproïque et où le sel d'acide valproïque est un sel de métal alcalin ou alcalino-terreux, **caractérisé en ce que** l'acide valproïque est réagi directement avec la quantité calculée du carbonate de métal alcalin ou carbonate de métal alcalino-terreux correspondant et/ ou avec la quantité calculée du bicarbonate de métal alcalin ou bicarbonate de métal alcalino-terreux correspondant, sans addition d'un solvant et à une température au-dessus du point de fusion de l'acide valproïque.

2. Procédé selon la revendication 1, **caractérisé en ce que** la température de réaction est comprise entre 50°C et 250°C, de préférence entre 70°C et 180°C, et où le dioxyde de carbone et l'eau, qui se forment durant la réaction, sont enlevés continuellement du mélange de réaction.

3. Procédé selon la revendication 1 ou 2, **caractérisé en ce que** un hydrate est préparé, comprenant l'addition de la quantité calculée d'eau au produit à la fin de la réaction.

4. Procédé selon l'une quelconque des revendications 1 à 3, **caractérisé en ce que** le sel d'acide valproïque sous forme de sel de métal alcalin est un sel de lithium, sodium, potassium ou rubidium, de préférence de sodium ou de potassium.

5. Procédé selon l'une quelconque des revendications 1 à 3, **caractérisé en ce que** le sel d'acide valproïque sous forme de sel de métal alcalino-terreux est un sel de magnésium, calcium, strontium ou baryum, de préférence de magnésium ou calcium.

6. Procédé selon l'une quelconque des revendications 1 à 5, **caractérisé en ce qu'**un composé de la formule générale (I):
[(CH₃CH₂CH₂)₂CH-C(O)OMe]ₘ. [(CH₃CH₂CH₂)₂ CH-C(O)OH]ₙ . xH₂O (I)
est préparé dans lequel
Me est Li⁺, Na⁺, K⁺, Rb⁺, Mg²⁺, Ca²⁺, Sr²⁺ ou Ba²⁺, de préférence Na⁺, K⁺, Mg²⁺, Ca²⁺,
m est un nombre entier entre 1 et 10, de préférence entre 1 et 6,
n est un nombre entier entre 1 et 9, de préférence entre 1 et 3,
et la proportion de m : n est de 1:0.05 à 6:1, de préférence de 1:1 à 5:3, de préférence de 1:1 ou 4:3 ou 2: 1, et
x est zéro, 1 ou 2, de préférence zéro ou 1.

7. Procédé selon l'une quelconque des revendications 1 à 6, **caractérisé en ce que** le composé 2-acide propylpentanoïque (3:2) sel de sodium est préparé.

8. Procédé selon l'une quelconque des revendications 1 à 6, **caractérisé en ce que** le composé 2-acide propylpentanoïque (4:3) sel de sodium monohydrate est préparé.
